# FASCICULE DE BREVET EUROPEEN

(11) **EP 0 628 393 B1**
(45) Date de publication et mention de la délivrance du brevet: **28.01.1998**
(21) Numéro de dépôt: 94400862.2
(22) Date de dépôt: 21.04.1994
(51) Int. Cl.: B29C 39/40, A45D 33/00

(54) **Procédé de moulage d'une composition de maquillage et produit de mquillage obtenu**
Verfahren zum Formen einer Schminkzusammensetzung und dadurch erhaltenes Schminkprodukt
Method for moulding a make-up composition and product obtained in such a way

(30) Priorité: 07.06.1993 FR 9306763
(43) Date de publication de la demande: 14.12.1994
(73) Titulaire: L'OREAL, 75008 Paris (FR)
(72) Inventeur: Joulia, Gérard, F-75019 Paris (FR)
(74) Mandataire: Peuscet, Jacques

(56) Documents cités:
- EP-A- 0 191 198
- EP-A- 0 528 705
- DE-A- 1 629 523
- FR-A- 1 520 770
- FR-A- 2 519 580
- US-A- 4 548 861
- DATABASE WPI Week 8628, Derwent Publications Ltd., London, GB; AN 86-179569 & JP-A-61 112 602 (HITACHI) 30 Mai 1986

## Description

La présente invention a pour objet un procédé de moulage d'une composition de maquillage par coulage dans un moule, sous forme fluide, et solidification de la composition, de façon à obtenir un produit moulé solide sous forme de pain ou de "cake", la composition pouvant ensuite être prélevée à partir du produit moulé à l'aide du doigt, d'un pinceau ou d'une houppette ; l'invention concerne aussi le produit obtenu par ce procédé.

La composition de maquillage à mouler peut être sous forme de pâte obtenue en mélangeant une phase particulaire solide soit avec une phase aqueuse, soit avec un agent liant, notamment une phase grasse, dans un solvant ; elle peut également être sous forme d'un produit à base de cire thermofusible ou d'un gel qui est coulé à l'état chaud. Selon la composition utilisée, la solidification se fait donc, soit par évaporation d'eau ou de solvant, soit par refroidissement, soit par réaction chimique.

Il est connu de couler la composition de maquillage dans un moule d'un certain volume comportant principalement une surface de moulage qui donne sa forme (plate, bombée ou munie de figures en relief ou en creux) à la surface de prélèvement de la composition et un fond, le plus souvent plat, muni d'une ou plusieurs ouvertures par lesquelles on coule la composition à mouler. Selon la composition de maquillage traitée, le phénomène de retrait au cours de la solidification peut être important. C'est le cas, notamment, pour les compositions décrites dans les brevets US-A-4 804 538, EP-A-165 137 et DE-A-3 327 001. Le pain moulé obtenu est ensuite conditionné dans un emballage, mais, à cause du retrait, il a des dimensions et une forme variables et il est difficile dans le conditionnement de le maintenir en place dans cet emballage et de le caler contre la surface rigide constituant le fond d'un emballage tel qu'un boîtier ; la surface rigide a pour fonction principale d'empêcher le pain moulé de se briser au cours des différentes manipulations et de le protéger mais le retrait supprime le calage et empêche le conditionnement de remplir sa fonction de façon satisfaisante. Si le pain moulé est mal calé, il a tendance à se briser au cours du stockage ; en effet, il présente par nature une grande friabilité car il doit être délitable pour permettre le prélèvement.

Pour éviter cet inconvénient, on a déjà proposé, dans EP-A-191 198 et dans EP-A-38 645, sur lesquels repose le préamble de la revendication 1, d'effectuer le coulage dans une coupelle reposant sur une surface de moulage, le fond de la coupelle étant muni de croisillons, d'armature ou d'autres dispositifs d'ancrage. La composition, en se solidifiant, s'accroche au fond de la coupelle et on conditionne ensuite un produit de moulage constitué par la composition solidifiée et sa coupelle associée. La coupelle sert de support à la composition solidifiée et ladite coupelle ayant des dimensions déterminées, le produit moulé est facile à maintenir en place et à caler dans un conditionnement rigide. Mais on a constaté, qu'après séchage ou refroidissement, il y a formation de fissures en raison d'un retrait de la composition par rapport à sa coupelle et à ses zones d'accrochage sur elle. Le produit vendu est, par conséquent, fragilisé et a tendance à éclater au cours des différentes manipulations ultérieures, notamment pendant son utilisation.

Il est donc souhaitable de définir un conditionnement de ce type de composition qui permette à la fois d'assurer le calage et le maintien de la composition solidifiée dans un conditionnement de protection écartant le risque de bris et de rendre possible, sans fragilisation du pain moulé, le retrait de la composition : il faut donc assurer simultanément deux fonctions.

Selon la présente invention, on a trouvé que les inconvénients susmentionnés sont évités en utilisant un fond de moule comportant une plaque de mousse à cellules ouvertes que l'on imprègne partiellement de la composition coulée. Le procédé selon l'invention permet d'assurer simultanément avec le produit obtenu les deux fonctions désirables ci-dessus définies.

La présente invention a donc pour objet un procédé de moulage d'une composition de maquillage dans un moule comportant une surface de moulage concave constituant la base du moule et un fond constituant l'élément supérieur du moule et obturant le volume délimité par ladite surface de moulage, la composition étant coulée dans ledit moule sous forme fluide et se solidifiant ensuite pour constituer un produit solide démoulable susceptible d'être conditionné, caractérisé par le fait que l'on coule ladite composition dans un moule dont le fond est au moins partiellement constitué par une plaque de mousse plastique à cellules ouvertes, de façon à imprégner partiellement ladite plaque de mousse avec ladite composition et qu'après solidification de ladite composition, on démoule, par enlèvement de la surface de moulage, un produit moulé, constitué par la composition solidifiée et par la plaque de mousse, qui lui est liée et qui en constitue le support.

On a constaté que, dans le procédé selon l'invention, on peut couler des compositions présentant un retrait important lors de leur solidification sans que le produit moulé obtenu soit fragile car le support de mousse, grâce à son élasticité, se conforme aux variations dimensionnelles de la composition solidifiée. Néanmoins, la mousse étant partiellement imprégnée par la composition, il se forme une liaison entre la plaque de mousse plastique et la composition lors de la solidification et ladite plaque de mousse constitue un support de la composition solidifiée, support qui, par lui-même, contribue au renforcement mécanique du pain moulé. On prépare donc un produit moulé constitué par l'ensemble (composition solidifiée-plaque de mousse). La fraction de la plaque de mousse non-imprégnée, plus particulièrement la couche de la plaque de mousse non imprégnée, qui a conservé son élasticité, peut être utilisée dans le conditionnement du produit moulé pour caler celui-ci sur une surface rigide en compensant les variations de dimensions de la composition solidifiée ; le calage est réalisé par une légère compression de la plaque de mousse. De plus, la composition solidifiée, étant maintenue dans l'emballage sur une surface élastique, est bien protégée contre les chocs qu'elle pourrait subir au cours des différentes manipulations. Une bonne conservation du produit moulé est donc assurée dès sa fabrication jusqu'à l'arrivée dans les mains de l'utilisateur.

Selon un premier mode de mise en oeuvre, on solidifie la composition après coulage par refroidissement ou par évaporation d'un solvant présent dans ladite composition. Selon un autre mode de mise en oeuvre, on coule une composition qui contient du plâtre (CaSO₄, 1/2 H₂O) et une quantité suffisante d'eau pour obtenir un mélange coulable, la solidification se produisant après coulage par prise du plâtre. Le procédé de moulage selon l'invention est alors particulièrement avantageux.

Selon l'invention, on peut couler la composition à travers la plaque de mousse ; on coule, de préférence, à travers une ouverture, avantageusement une ouverture centrale, ménagée dans la plaque de mousse. La dimension de cette ouverture est fonction de la dimension du moule et de la composition coulée.

De préférence, on comprime légèrement la plaque de mousse au stade de solidification, avantageusement à l'aide d'une platine rigide : cette platine rigide est, avantageusement, munie d'ouvertures pour faciliter l'évaporation lorsque la solidification se fait par évaporation.

La plaque de mousse utilisée selon l'invention est constituée, comme indiqué ci-dessus, d'une mousse plastique à cellules ouvertes. Cette mousse est choisie de façon à ne pratiquement pas gonfler en présence des différents ingrédients de la composition de maquillage, tels que eau, huile et corps gras, et à être susceptible d'être imprégnée par ladite composition. On utilise avantageusement une mousse ayant une structure cellulaire telle que le volume occupé par les parois des cellules ne représente pas plus de 3 % du volume total de la mousse. Avantageusement, la mousse a une structure cellulaire homogène, c'est-à-dire constituant un réseau tridimensionnel. Des mousses plastiques convenables sont, par exemple, les mousses de polyuréthanne réticulé, en particulier celles commercialisées sous les dénominations "BULPREN S 20", "BULPREN S 30" et "FILTREN S 2120" par la société "RECTICEL".

L'épaisseur de la plaque de mousse utilisée est fonction de la dimension du moule et de la composition coulée. Une épaisseur convenable est généralement de l'ordre de 3 à 6 mm.

La présente invention a également pour objet le produit de maquillage moulé obtenu par le procédé précédemment défini.

Ce produit est, de préférence, conditionné, après démoulage, dans un présentoir rigide constitué d'un élément qui supporte la plaque de mousse et d'une bague qui entoure le produit moulé et est rendue solidaire dudit élément.

Dans une première variante, l'élément est une embase, qui coopère avec une bague prenant appui sur la composition solidifiée pour maintenir le produit moulé contre l'embase, ladite embase débordant latéralement par rapport à la plaque de mousse.

Dans une deuxième variante, l'élément est une platine et la bague comporte un moyen de solidarisation susceptible d'assurer périphériquement, de façon amovible, sa liaison avec une capsule pour constituer avec elle une surface de moulage. De plus, ladite platine comporte, de préférence, au moins une ouverture.

On peut avantageusement prévoir que le produit selon l'invention contienne du plâtre hydraté (CaSO₄, 2 H₂O), de préférence dans une proportion pondérale comprise entre 10 et 50 %.

La description donnée ci-après, à titre purement illustratif et non limitatif, permettra de mieux comprendre l'invention en se référant au dessin annexé.

Sur ce dessin :
- la figure 1 est un schéma des différentes étapes (a à f) d'une première variante du procédé de moulage selon l'invention ;
- la figure 2 représente une vue éclatée d'un moule utilisé selon une deuxième variante du procédé selon l'invention ; et
- la figure 3 représente les différentes étapes d'utilisation du moule illustré sur la figure 2.

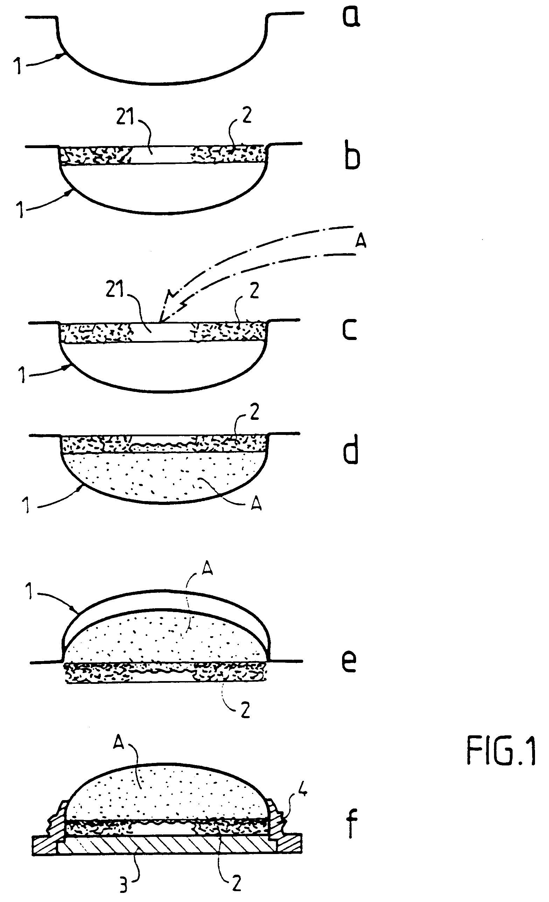

Dans le schéma représenté sur la figure 1 :
- à l'étape a, on met en place une coupelle 1 thermoformée, qui constitue la surface concave de moulage de la composition de maquillage ;
- à l'étape b, on met en place, dans l'ouverture libre de la coupelle 1, une plaque de mousse 2 munie d'une ouverture centrale 21, cette plaque de mousse 2 constituant le fond du moule ;
- à l'étape c, on verse la composition cosmétique A par l'ouverture 21, comme indiqué par la flèche, et on remplit totalement le moule de façon à imprégner de composition A, la couche inférieure de la plaque de mousse 2, tout en laissant libre la couche supérieure de ladite plaque de mousse, couche supérieure qui conserve, par conséquent, son élasticité ;
- à l'étape d, on laisse la composition A se solidifier ; il se forme alors une liaison mécanique entre la plaque de mousse 2 et la composition A solidifiée ;
- à l'étape e, on démoule le produit moulé constitué par une pastille bombée de la composition A solidifiée et par la plaque de mousse 2 qui en constitue le support ;
- l'étape f est l'étape de conditionnement du produit moulé ; on fixe le produit moulé sur une embase rigide 3 à l'aide d'une bague 4 en comprimant légèrement la plaque de mousse 2 ; la bague 4 prend appui sur la pastille de composition A et l'embase 3 est rendue solidaire de la bague 4 pour maintenir la compression de la mousse. De cette façon, la plaque de mousse 2, grâce à son élasticité, permet, par rapport à l'embase 3 et à la bague 4, de compenser les déformations de la pastille de composition A solidifiée dues au retrait au cours de la solidification et après celle-ci. La pastille de composition A est donc bien maintenue et bien calée sur l'embase rigide 3 sans que l'on risque de fissurations de la composition solidifiée.

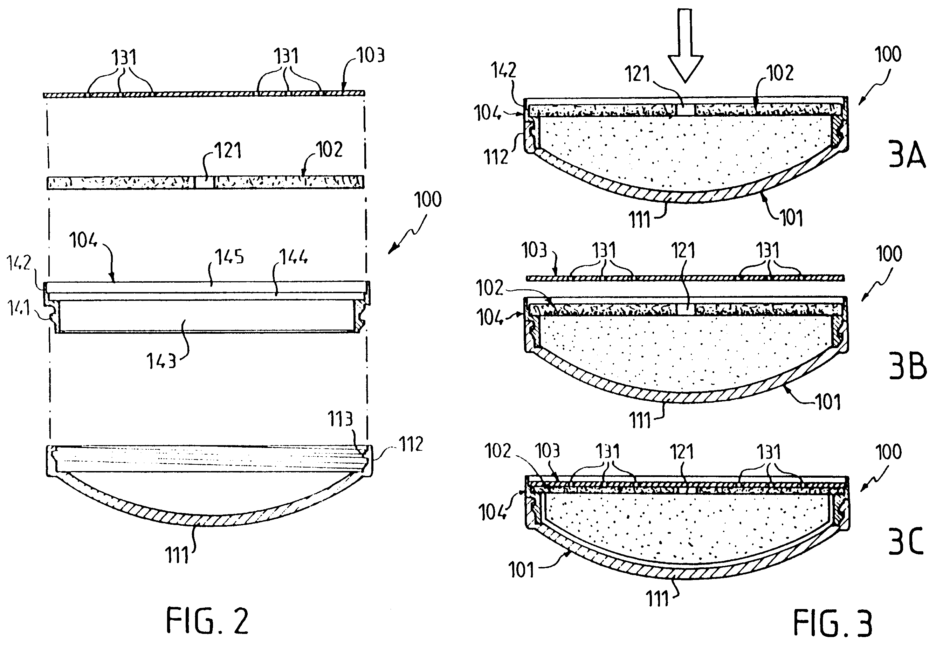

La figure 2 représente en vue éclatée les différentes pièces constituant un moule 100 pour la mise en oeuvre d'une deuxième variante du procédé selon l'invention, variante que l'on préfère car elle permet de mouler directement dans l'emballage du produit moulé. Il se compose, en premier lieu, d'une surface de moulage en forme de cuvette référencée 101 dans son ensemble, comme on le voit sur la figure 3A, en second lieu, d'une plaque de mousse 102 en forme de disque circulaire et, en troisième lieu, d'une platine rigide 103 également en forme de disque circulaire, de même surface que celle de la plaque de mousse 102. La surface de moulage 101 se compose, en premier lieu, d'une capsule 111 concave sur le bord de laquelle est conformée une jupe cylindrique 112 munie d'un pas de vis interne 113 et, en deuxième lieu, d'une bague amovible 104 munie d'un pas de vis externe 141 et susceptible de s'assembler par vissage sur la jupe 112. Le pas de vis 141 est situé un peu en retrait d'une surface cylindrique 142 qui vient dans le prolongement de la surface externe de la jupe 112, lorsque la bague 104 est vissée sur la capsule 111.

La surface intérieure de la bague 104 définit trois zones de diamètre croissant séparées par des épaulements : une zone de plus faible diamètre 143 située du côté de la partie concave de la capsule 111, lorsque la bague 104 est vissée sur ladite capsule, une zone de plus grand diamètre 144 ayant un diamètre égal, au jeu nécessaire près, au diamètre externe de la plaque de mousse 102 et une hauteur inférieure à l'épaisseur de la plaque de mousse 102 et, enfin, une zone 145 de diamètre encore plus élevé et de hauteur voisine de l'épaisseur de la platine rigide 103. La plaque de mousse 102 est munie d'une ouverture centrale 121 et la platine rigide 103 est pourvue de plusieurs ouvertures 131.

Comme illustré sur la figure 3A, on monte la bague sur la capsule 111, on dispose la plaque de mousse 102 dans la zone 144 de la bague 104 vissée sur la jupe 112 de la capsule 111, pour constituer la surface de moulage 101. On introduit la composition cosmétique par l'ouverture centrale 121 de la plaque de mousse 102 jusqu'à ce que la composition cosmétique remplisse la zone 144 de la bague et vienne imprégner la mousse sur une fraction de son épaisseur : généralement, la moitié de son épaisseur ; on obtient ainsi, après solidification, une liaison entre la composition solidifiée et la plaque de mousse 102. On monte ensuite à force, dans la zone 145 de la bague 104, la platine 103, qui vient légèrement écraser la plaque de mousse 102 (voir figures 3B et 3C) sur l'épaulement entre les zones 144 et 145 et assure donc la fixation de la plaque de mousse contre la composition coulée. Cet écrasement peut être effectué avant solidification totale, auquel cas il participe à l'imprégnation partielle de la plaque de mousse 102, ou après solidification totale.

On laisse ensuite sécher la composition cosmétique. Les cellules de la mousse et les ouvertures 131 ménagées dans la plaque 103 permettent l'évaporation du solvant lorsque la composition cosmétique se solidifie par évaporation et, par conséquent, séchage final.

Après séchage, on désolidarise la capsule 111 de la bague 104 et on obtient ainsi un ensemble 103, 102, 104 qui peut être directement commercialisé ; la commercialisation peut aussi intervenir avec la capsule 111 qui sert alors de couvercle pour protéger la composition. La composition cosmétique solidifiée est maintenue sur la platine 103 grâce à la plaque de mousse 102 bloquée entre la bague 104 et la platine 103. La rigidité de la platine 103 évite tout risque de bris du pain que constitue la composition solidifiée. Néanmoins, la composition solidifiée peut subir un retrait sans se fissurer en raison de l'élasticité de la plaque de mousse 102.

Il suffit à l'utilisateur de dévisser le couvercle 101 pour dégager la surface de prélèvement de la composition de maquillage moulée et prélever la composition de maquillage à l'aide d'un pinceau ou d'une houppette.

Deux exemples de mise en oeuvre du procédé selon l'invention sont donnés ci-après (les quantités sont indiquées en g).

### EXEMPLE 1 : Préparation d'un fard à paupières

On prépare une pâte ayant la formulation suivante :

| **Phase A** | |
|---|---|
| - Talc | 9,7 |
| - Oxyde de chrome | 1,8 |
| - Bleu outremer | 0,7 |
| - Stéarate de zinc | 0,7 |
| - Mica | 5,3 |
| - Amidon | 1,7 |
| - Micatitane | 10,5 |
| - Microsphères creuses (cavité unique) en copolymère de chlorure de vinylidène-acrylonitrile (masse volumique 0,02 g/cm³) commercialisées sous la dénomination "EXPANCEL 551 DE" par la société "KEMANORD PLAST" | 1,4 |

| **Phase B** | |
|---|---|
| - Copolymère polyvinylpyrrolidone hexadécène | 0,2 |
| - Huile de jojoba | 0,5 |
| - Myristate d'isopropyle | 0,7 |
| - Lanoline | 0,4 |
| - Huile d'amande douce | 1,2 |

| **Conservateurs** | |
|---|---|
| - Butylhydroxytoluène | 0,015 |
| - Butylhydroxyanisole | 0,015 |
| - Parahydroxybenzoate de propyle | 0,1 |

| **Solvant** | |
|---|---|
| - Cyclométhicone | 65 |

Dans un premier temps, on mélange entre eux les constituants de la phase B. On introduit par ailleurs, dans un mélangeur, les constituants de la phase A, puis le solvant et on homogénéise le tout. Puis, la phase B où on a introduit les conservateurs est introduite à son tour dans le mélangeur et on mélange à nouveau jusqu'à obtention d'une pâte homogène.

La pâte obtenue est coulée, à température ambiante, dans le moule concave représenté sur la figure 1. Ce moule est réalisé en polypropylène injecté et a un diamètre de 60 mm et une hauteur de 20 mm. Le fond est constitué par une plaque de mousse 2 de "BULPREN S 20" ayant une épaisseur de 6 mm et muni d'une ouverture centrale 21 de 15 mm de diamètre ; la pâte est introduite par ladite ouverture centrale 21. Les moules sont ensuite placés en étuve à 40°C pendant 55 heures. On démoule ensuite un produit constitué par la composition solidifiée, solidarisée avec la plaque de mousse 2 ; on fixe ensuite le produit démoulé sur l'embase rigide 3, de même dimension que la plaque de mousse 2, à l'aide de la bague 4, de façon à laisser libre la surface de prélèvement. On peut ensuite adapter un couvercle sur la bague 4 ou introduire l'ensemble (composition solidifiée liée à la plaque de mousse 2, bague 4 et embase 3) dans un boîtier.

La pastille bombée de fard à paupières bleu-vert obtenue présente une surface lisse et homogène pouvant être prélevée facilement au doigt ou avec un pinceau.

### EXEMPLE 2 : Fard à joues

On prépare un mélange pulvérulent ayant la formulation suivante :

| | |
|---|---|
| - Plâtre (CaSO₄, 1/2 H₂O) | 25 |
| - Talc | 25 |
| - Talc enrobé de lauroyl lysine commercialisé sous la dénomination "EP 90025 TALC TREATED" par la société "MEARL" | 10 |
| - Microsphères creuses (cavité unique) en copolymère de chlorure de vinylidène-acrylonitrile (masse volumique 0,02 g/cm³) commercialisées sous la dénomination "EXPANCEL 551 DE" par la société "KEMANORD PLAST" | 5 |
| - Mica | 24 |
| - Carbonate de calcium | 5 |
| - Dioxyde de titane | 2 |
| - Oxyde de fer rouge | 3,5 |
| - Oxyde de fer noir | 0,5 |
| et une phase aqueuse ayant la composition suivante : | |
| - Eau | 120 |
| - Tensioactif commercialisé sous la dénomination "GLUCQUAT 100" par la société "AMERCHOL" | 4,5 |
| - Conservateur | 0,1 |

On mélange le mélange pulvérulent et la phase aqueuse dans un mélangeur muni d'une agitation lente pendant 5 minutes.

On introduit le mélange obtenu dans le moule, représenté sur la figure 2 ; ce moule a des dimensions identiques au moule de l'exemple 1. La surface de moulage 101 associe à la capsule 111 une bague 104 comportant trois zones 143, 144, 145 ayant respectivement les hauteurs suivantes : 7 ; 2 ; 2,5 mm et les diamètres suivants : 66 ; 70 ; 71,5 mm.

On introduit à force, dans la zone 144, un disque d'une mousse vendue sous la dénomination commerciale "FILTREN S 2120" ayant une épaisseur de 3 mm comportant une ouverture circulaire centrale 121 de 20 mm de diamètre. On verse la composition fluide par l'ouverture 121. On imprègne de solution une épaisseur de mousse d'environ 1,5 mm. Environ 30 minutes après le remplissage du moule, on introduit à force, dans la bague, une platine 103 ayant, au jeu nécessaire près, le diamètre de la zone 145, ce qui comprime la couche de mousse sur l'épaulement entre les zones 143 et 144 sur 2 mm et fixe la plaque de mousse sur la platine 103. La platine comporte 24 trous régulièrement répartis ayant un diamètre de 1 mm pour permettre le séchage final. Le moule contenant le produit solidifié est stocké tel quel pendant environ trois jours, ce qui permet à la composition de sécher, car la vapeur d'eau peut s'échapper par les ouvertures 131 de la platine 103. Le produit est ensuite commercialisé tel quel. Il suffit à l'utilisateur de dévisser la capsule 111 pour dégager la surface de prélèvement et de prélever la composition de maquillage à l'aide d'une houppette ou d'un pinceau.

## Revendications

1. Procédé de moulage d'une composition de maquillage dans un moule comportant une surface de moulage concave constituant la base du moule et un fond constituant l'élément supérieur du moule et obturant le volume délimité par ladite surface de moulage, la composition étant coulée dans ledit moule sous forme fluide et se solidifiant ensuite pour constituer un produit solide démoulable susceptible d'être conditionné, caractérisé par le fait que l'on coule ladite composition dans un moule dont le fond est au moins partiellement constitué par une plaque de mousse plastique à cellules ouvertes, de façon à imprégner partiellement ladite plaque de mousse (2, 102) avec ladite composition et qu'après solidification de ladite composition, on démoule, par enlèvement de la surface de moulage, un produit moulé, constitué par la composition solidifiée et par la plaque de mousse, qui lui est liée et qui en constitue le support.

2. Procédé selon la revendication 1, caractérisé par le fait qu'après coulage, on solidifie la composition par refroidissement ou par évaporation d'un solvant présent dans ladite composition.

3. Procédé selon l'une des revendications 1 ou 2, caractérisé par le fait que l'on coule une composition, qui contient du plâtre (CaSO₄, 1/2 H₂O) et une quantité suffisante d'eau pour obtenir un mélange coulable, la solidification se produisant après coulage en raison de la prise du plâtre.

4. Procédé selon l'une des revendications 1 à 3, caractérisé par le fait qu'on coule la composition de maquillage à travers la plaque de mousse (2, 102) préalablement mise en place pour constituer l'élément supérieur du moule.

5. Procédé selon la revendication 4, caractérisé par le fait qu'on coule la composition de maquillage à travers une ouverture centrale (21) ménagée dans la plaque de mousse (2).

6. Procédé selon l'une des revendications 1 à 5, caractérisé par le fait qu'après coulage de la composition dans le moule, on comprime la plaque de mousse (102) à l'aide d'une platine rigide (103) pour l'appuyer contre la composition moulée.

7. Procédé selon la revendication 6, caractérisé par le fait qu'on comprime la plaque de mousse (2, 102) au cours de la solidification de la composition moulée.

8. Procédé selon la revendication 7, caractérisé par le fait qu'on munit la platine rigide (103) d'au moins une ouverture (131).

9. Procédé selon l'une des revendications 1 à 8, caractérisé par le fait qu'on choisit pour constituer la plaque de mousse (2, 102) une structure cellulaire telle que le volume occupé par les parois des cellules ne représente pas plus de 3 % du volume total de la mousse.

10. Procédé selon l'une des revendications 1 à 9, caractérisé par le fait qu'on choisit, pour constituer la plaque de mousse, une mousse de polyuréthanne réticulé.

11. Procédé selon l'une des revendications 1 à 10, caractérisé par le fait qu'on choisit une plaque de mousse ayant une épaisseur comprise entre 3 et 6 mm.

12. Produit de maquillage moulé obtenu par le procédé selon l'une des revendications 1 à 11.

13. Produit selon la revendication 12 obtenu par le procédé selon la revendication 3, caractérisé par le fait qu'il contient du plâtre hydraté (CaSO₄, 2 H₂O).

14. Produit selon la revendication 13, caractérisé par le fait qu'il contient de 10 à 50 % en poids de plâtre hydraté.

15. Produit selon l'une des revendications 12 à 14, caractérisé par le fait qu'il est conditionné, après démoulage, dans un présentoir rigide.

16. Produit selon la revendication 15, caractérisé par le fait que le présentoir rigide est constitué d'un élément (3, 103), qui supporte la plaque de mousse (2, 102), et d'une bague (4, 104) qui entoure le produit moulé et est rendue solidaire dudit élément (3, 103).

17. Produit selon la revendication 16, caractérisé par le fait que l'élément est une embase (3), qui coopère avec une bague (4) prenant appui sur la composition solidifiée pour maintenir le produit moulé contre l'embase, ladite embase (3) débordant latéralement par rapport à la plaque de mousse (2).

18. Produit selon la revendication 16, caractérisé par le fait que l'élément est une platine (103) et que la bague (104) comporte un moyen de solidarisation susceptible d'assurer périphériquement, de façon amovible, sa liaison avec une capsule (111) pour constituer avec elle une surface de moulage.

19. Produit selon la revendication 18, caractérisé par le fait que la platine (103) comporte au moins une ouverture (131).

## Claims

1. Process for moulding a make-up composition in a mould comprising a concave moulding surface forming the base of the mould and a bottom forming the upper component of the mould and shutting off the space bounded by the said moulding surface, the composition being cast in the said mould in fluid form and subsequently solidifying to form a demouldable solid product capable of being packaged, characterized in that the said composition is cast in a mould the bottom of which is at least partly formed by a sheet of open-cell plastic foam so as partly to impregnate the said foam sheet (2, 102) with the said composition and in that after solidification of the said composition a moulded product, consisting of the solidified composition and of the foam sheet which is bonded to it and which forms its support, is demoulded by removing the moulding surface.

2. Process according to Claim 1, characterized in that, after casting, the composition is solidified by cooling or by evaporation of a solvent present in the said composition.

3. Process according to either of Claims 1 and 2, characterized in that a composition is cast which contains plaster (CaS₄.½H₂O) and a sufficient quantity of water to obtain a pourable mixture, the solidification taking place after casting as a result of the setting of the plaster.

4. Process according to one of Claims 1 to 3, characterized in that the make-up composition is cast through the foam sheet (2, 102) put in place beforehand to form the upper component of the mould.

5. Process according to Claim 4, characterized in that the make-up composition is cast through a central opening (21) made in the foam sheet (2).

6. Process according to one of Claims 1 to 5, characterized in that, after casting of the composition in the mould, the foam sheet (102) is compressed with the aid of a rigid plate (103) to press it against the moulded composition.

7. Process according to Claim 6, characterized in that the foam sheet (2, 102) is compressed during the solidification of the moulded composition.

8. Process according to Claim 7, characterized in that the rigid plate (103) is provided with at least one opening (131).

9. Process according to one of Claims 1 to 8, characterized in that a cell structure such that the volume occupied by the walls of the cells does not represent more than 3 % of the total volume of the foam is chosen to form the foam sheet (2, 102).

10. Process according to one of Claims 1 to 9, characterized in that a crosslinked polyurethane foam is chosen to form the foam sheet.

11. Process according to one of Claims 1 to 10, characterized in that a foam sheet which has a thickness of between 3 and 6 mm is chosen.

12. Moulded make-up product obtained by the process according to one of Claims 1 to 11.

13. Product according to Claim 12, obtained by the process according to Claim 3, characterized in that it contains hydrated plaster (CaSO₄.2H₂O).

14. Product according to Claim 13, characterized in that it contains from 10 to 50 % by weight of hydrated plaster.

15. Product according to one of Claims 12 to 14, characterized in that, after demoulding, it is packaged in a rigid display case.

16. Product according to Claim 15, characterized in that the rigid display case consists of a component (3, 103) which supports the foam sheet (2, 102) and of a ring (4, 104) which surrounds the moulded product and is integrally attached to the said component (3, 103).

17. Product according to Claim 16, characterized in that the component is a base (3) which interacts with a ring (4) which bears on the solidified composition to hold the moulded product against the base, the said base (3) overlapping laterally in relation to the foam sheet (2).

18. Product according to Claim 16, characterized in that the component is a plate (103) and that the ring (104) comprises a means for integral fastening capable of ensuring peripherally, in a removable manner, its bonding to a cap (111) to form a moulding surface with it.

19. Product according to Claim 18, characterized in that the plate (103) comprises at least one opening (131).

## Patentansprüche

1. Verfahren zur Formung einer Schminkzusammensetzung in einer Form, die eine ihre Basis bildende konkave Formfläche und einen ihr oberes Element bildenden Boden aufweist, der das von der Formfläche abgegrenzte Volumen schließt, wobei die Zusammensetzung in flüssigem Zustand in die Form gegossen wird und anschließend erstarrt, um ein der Form entnehmbares und verpackbares festes Produkt zu bilden, dadurch gekennzeichnet, daß man die Zusammensetzung in eine Form gießt, deren Boden mindestens teilweise von einer Kunststoffschaumplatte mit offenen Zellen gebildet ist, so daß die Schaumstoffplatte (2, 102) teilweise mit der Zusammensetzung getränkt wird, und daß man nach dem Erstarren der Zusammensetzung durch Entfernung der Formfläche der Form ein geformtes Produkt entnimmt, das aus der erstarrten Zusammensetzung und aus der mit ihr verbundenen und ihren Träger bildenden Schaumstoffplatte besteht.

2. Verfahren nach Anspruch 1, dadurch gekennzeichnet, daß man die Zusammensetzung nach dem Gießen durch Abkühlung oder durch Abdampfen eines in der Zusammensetzung enthaltenen Lösungsmittels erstarren läßt.

3. Verfahren nach einem der Ansprüche 1 oder 2, dadurch gekennzeichnet, daß man eine Zusammensetzung gießt, die Gips (CaSO₄, 1/2H₂O) und eine zum Erhalten einer gießfähigen Mischung ausreichende Menge Wasser enthält, wobei die Erstarrung nach dem Gießen durch das Abbinden des Gipses erreicht wird.

4. Verfahren nach einem der Ansprüche 1 bis 3, dadurch gekennzeichnet, daß man die Schminkzusammensetzung durch die Schaumstoffplatte (2, 102) gießt, die zuvor eingesetzt wurde, um das obere Element der Form zu bilden.

5. Verfahren nach Anspruch 4, dadurch gekennzeichnet, daß man die Schminkzusammensetzung durch eine in der Schaumstoffplatte (2) vorgesehene zentrale Öffnung (21) gießt.

6. Verfahren nach einem der Ansprüche 1 bis 5, dadurch gekennzeichnet, daß man nach dem Eingießen der Zusammensetzung in die Form die Schaumstoffplatte (102) mit Hilfe einer starren Platte (103) komprimiert, um sie an die geformte Zusammensetzung anzudrücken.

7. Verfahren nach Anspruch 6, dadurch gekennzeichnet, daß man die Schaumstoffplatte (2, 102) während des Erstarrens der geformten Zusammensetzung komprimiert.

8. Verfahren nach Anspruch 7, dadurch gekennzeichnet, daß man die starre Platte (103) mit mindestens einer Öffnung (131) versieht.

9. Verfahren nach einem der Ansprüche 1 bis 8, dadurch gekennzeichnet, daß man zur Bildung der Schaumstoffplatte (2, 102) eine Zellstruktur wählt, bei der das von den Zellwänden eingenommene Volumen nicht mehr als 3 % des Gesamtvolumens des Schaumstoffs ausmacht.

10. Verfahren nach einem der Ansprüche 1 bis 9, dadurch gekennzeichnet, daß man zur Bildung der Schaumstoffplatte einen vernetzten Polyurethanschaum wählt.

11. Verfahren nach einem der Ansprüche 1 bis 10, dadurch gekennzeichnet, daß man eine Schaumstoffplatte wählt, die eine Dicke von 3 bis 6 mm aufweist.

12. Geformtes Schminkprodukt, das in dem Verfahren nach einem der Ansprüche 1 bis 11 erhalten wurde.

13. Produkt nach Anspruch 12, das in dem Verfahren nach Anspruch 3 erhalten wurde, dadurch gekennzeichnet, daß es hydratisierten Gips (CaSO₄, 2H₂O) enthält.

14. Produkt nach Anspruch 13, dadurch gekennzeichnet, daß es 10 bis 50 Gew.-% hydratisierten Gips enthält.

15. Produkt nach einem der Ansprüche 12 bis 14, dadurch gekennzeichnet, daß es nach der Formabnahme in einem starren Behälter verpackt wird.

16. Produkt nach Anspruch 15, dadurch gekennzeichnet, daß der starre Behälter aus einem Element (3, 103), das die Schaumstoffplatte (2, 102) trägt, und aus einem Ring (4, 104) besteht, der das geformte Produkt umgibt und mit dem Element (3, 103) fest verbunden ist.

17. Produkt nach Anspruch 16, dadurch gekennzeichnet, daß das Element ein Sockel (3) ist, der mit einem Ring (4) zusammenwirkt, der sich auf die erstarrte Zusammensetzung aufstützt, um das geformte Produkt an den Sockel angedrückt zu halten, wobei der Sockel (3) seitlich an der Schaumstoffplatte (2) vorsteht.

18. Produkt nach Anspruch 16, dadurch gekennzeichnet, daß das Element eine Platine (103) ist und daß der Ring (104) eine Befestigungseinrichtung aufweist, die seine lösbare umfängliche Verbindung mit einer Kapsel (111) gestattet, um mit ihr eine Formfläche zu bilden.

19. Produkt nach Anspruch 18, dadurch gekennzeichnet, daß die Platine (103) mindestens eine Öffnung (131) aufweist.
